(19) Europäisches Patentamt / European Patent Office / Office européen des brevets

(11) **EP 3 257 947 B1**

(12) **FASCICULE DE BREVET EUROPEEN**

(45) Date de publication et mention
de la délivrance du brevet:
**19.12.2018 Bulletin 2018/51**

(51) Int Cl.:
**C12Q 1/04** (2006.01)

(21) Numéro de dépôt: **16174717.5**

(22) Date de dépôt: **16.06.2016**

(54) **PROCEDE ET SYSTEME D'IDENTIFICATION DU TYPE DE GRAM D'UNE BACTERIE**

VERFAHREN UND SYSTEM ZUR IDENTIFIZIERUNG DES GRAMTYPS EINER BAKTERIE

METHOD AND SYSTEM FOR IDENTIFYING THE GRAM TYPE OF A BACTERIUM

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(43) Date de publication de la demande:
**20.12.2017 Bulletin 2017/51**

(73) Titulaire: **Biomérieux**
**69280 Marcy L'etoile (FR)**

(72) Inventeurs:
• **LEROUX, Denis**
**01600 Trevoux (FR)**
• **LALOUM, Eric**
**75005 Paris (FR)**
• **MAHE, Pierre**
**38250 Lans en Vercors (FR)**
• **MIDAHUEN, Rony**
**38600 Fontaine (FR)**
• **BARLAS, Philippine**
**38500 La Buisse (FR)**

(56) Documents cités:
**WO-A2-2010/077304**

• **BOSOON PARK ET AL: "Hyperspectral microscope imaging methods to classify gram-positive and gram-negative foodborne pathogenic bacteria", TRANSACTIONS OF THE AMERICAN SOCIETY OF AGRICULTURAL ENGINEERS, AMERICAN SOCIETY OF AGRICULTURAL ENGINEERS. ST.JOSEPH, MI, US, vol. 58, no. 1, 1 janvier 2015 (2015-01-01), pages 5-16, XP008182213, ISSN: 0001-2351, DOI: 10.13031/TRANS.58.10832**
• **LUNA-PINEDA T; SOTO-FELICIANO K; DE LA CRUZ-MONTOYA E; LONDONO L C P; RIOS-VELAZQUEZ C; HERNANDEZ-RIVERA S P: "Spectroscopic characterization of biological agents using FTIR, normal Raman and surface-enhanced Raman scattering", PROCEEDINGS OF THE SPIE - THE INTERNATIONAL SOCIETY FOR OPTICAL ENGINEERING, vol. 6554, 9 avril 2007 (2007-04-09), - 27 avril 2007 (2007-04-27), pages 1-11, XP040239960, USA ISSN: 0277-786X, DOI: 10.1117/12.720338**
• **GUILLEMOT MATHILDE ET AL: "Hyperspectral imaging for presumptive identification of bacterial colonies on solid chromogenic culture media", OPTICAL SENSING II, SPIE, 1000 20TH ST. BELLINGHAM WA 98225-6705 USA, vol. 9887, 27 avril 2016 (2016-04-27), pages 98873L-98873L, XP060069290, ISSN: 0277-786X, DOI: 10.1117/12.2229761 ISBN: 978-1-62841-971-9**

**Description**

**DOMAINE DE L'INVENTION**

L'invention a trait au domaine de l'analyse microbiologique, et en particulier de l'identification du type de Gram d'une bactérie.

De manière avantageuse, l'invention s'applique à l'analyse d'une image hyperspectrale ou multispectrale d'une colonie bactérienne ayant poussé dans un milieu nutritif non chromogène, non fluorogène et dépourvu de colorant.

**ETAT DE LA TECHNIQUE**

La classification de Gram d'une souche de bactérie permet de caractériser la paroi celle-ci, par exemple son pourcentage en peptidoglycane, et est utilisé dans la taxonomie des bactéries ou pour évaluer leur sensibilité aux antibiotiques. On distingue ainsi deux types de bactérie, à savoir les bactéries à Gram « positif » et les bactéries à Gram « négatif ». La connaissance du type de Gram d'une souche bactérienne permet par exemple de sélectionner des tests appropriés pour l'identification de la souche ou la réalisation d'un antibiogramme, e.g. le choix d'une carte appropriée pour un test d'antibiogramme réalisé par l'automate Vitek© 2 commercialisé par la Demanderesse.

Historiquement, le type de Gram d'une souche bactérienne était déterminé par une technique manuelle dite de « coloration de Gram », qui comprend un nombre important d'étapes manuelles (fixation, coloration, mordançage, lavage, sur-coloration...), et donc longue à mettre en oeuvre. Diverses techniques ont donc été mises au point pour automatiser la détection du type de Gram des bactéries, afin notamment de traiter un nombre important d'échantillons. Toutefois, ces techniques continuent pour l'essentiel à modifier la réponse électromagnétique des bactéries ou de leur milieu pour rendre leur Gram aisément observable.

Par exemple, un premier type de technique consiste à automatiser la coloration de la membrane bactérienne sur des lamelles de microscope, mais l'étape de décision finale concernant le type de Gram est toujours réalisée par un technicien qui observe les lamelles au microscope. Ce type de technique n'est donc pas entièrement automatisée, et par ailleurs difficilement automatisable. En effet, la différence de couleurs entre les bactéries de Gram positif et les bactéries de Gram négatif peut être subtil, expliquant pourquoi l'intervention d'un technicien de laboratoire est encore nécessaire.

Un deuxième type de technique consiste à mettre des bactéries en présence d'un substrat qui se dégrade par une réaction enzymatique initiée par les peptidoglycanes des membranes des bactéries. Cette réaction produit des chromophores ou des fluorophores dont la concentration est une indication du Gram. On parle usuellement de « marquage » chromogène ou fluorogène des bactéries. Si ce type de techniques de l'état de l'art est automatisable, par exemple en mesurant l'intensité lumineuse des chromophores/fluorophores à l'aide d'un dispositif approprié (e.g. spectromètre/fluoromètre) puis en comparant informatiquement l'intensité mesurée à des valeurs de seuils prédéfinies, elles supposent néanmoins la conception de substrats chromophores ou fluorogènes particuliers, souvent couteux.

En outre, quelle que soit la technique employée, les bactéries subissent une modification de leur état naturel (e.g. elles comprennent des colorants, ont fixé des marqueurs chromogènes ou fluorescentes, etc.), et ne sont donc plus utilisables pour des tests de caractérisations ultérieurs (e.g. la détermination d'un antibioGramme).

Le document « Hyperspectral microscope imaging methods to classify gram positive and gram-negative foodborn pathogenic bacteria » de Bosoon Park et al (Bosoon P et al., Transactions of the ASABE 2015; 58(1): 5-16) décrit un procédé de détection du type de gram d'une bactérie dans la gamme de longueur d'onde 450nm-800nm. Le document WO2010/077304 décrit la détection du type de gram d'une bactérie par analyse de sa fluorescence intrinsèque. Le document « Spectroscopic characterization of biological agents using FTIR, normal Raman and surface-enhanced Raman scattering » de Luna-Pineda T. et al (Luna-Pineda T et al., Proc of SPIE 2007; 6554: 65540K1-11) décrit la détection du type de gram d'une bactérie se basant sur la mesure de SERS (« Surface Enhanced Raman Scattering »). Le document « Hyperspectral imaging for presumptive identification of bacterial colonies on solide chromogenic culture média » de Guillemot M. et al (Guillemot M et al., Proc of SPIE 2016; 9887: 98873L1-12) décrit une possibilité de détection du type de Gram d'une bactérie par l'analyse d'une image hyperspectrale d'une colonie de ladite bactérie.

**EXPOSE DE L'INVENTION**

Le but de la présente invention est de proposer un procédé de détermination du type de Gram d'une souche de bactérie qui soit automatique et qui ne nécessite pas de marquer ou de colorer la bactérie pour de déterminer son Gram.

A cet effet, l'invention a pour objet un procédé de détection du type de Gram d'une souche bactérienne, comprenant :

- l'éclairage dans la gamme de longueurs d'onde 415nm-440nm d'au moins une bactérie de ladite souche ayant une réponse électromagnétique naturelle dans ladite gamme;

- l'acquisition, dans la gamme 415nm-440nm, d'une intensité lumineuse réfléchie par, ou transmise au travers de, ladite bactérie éclairée; et
- la détermination du type de Gram de la souche bactérienne en fonction de l'intensité lumineuse acquise dans la gamme 415nm-440nm.

Par réponse « électromagnétique naturelle », on entend au sens de l'invention que la bactérie n'est pas modifiée à l'aide d'éléments (colorant, chromogène, fluorogène, etc.) qui modifient sa réponse électromagnétique à un éclairage au moins dans la gamme de longueurs d'onde d'intérêt. Par exemple, une colonie de la souche est cultivée dans un milieu nutritif non coloré, non chromogène et non fluorescent et l'éclairage/acquisition se fait directement sur la colonie encore présente dans son milieu.

En d'autres termes, les inventeurs ont découvert une gamme de longueurs d'onde dans laquelle une bactérie a « naturellement » une signature électromagnétique caractéristique de son type de Gram. Le procédé selon l'invention consiste ainsi à mesurer cette signature puis à en extraire le type de Gram. Ainsi, il n'est pas nécessaire d'utiliser un substrat chromogène ou fluorogène ou des colorants. Par ailleurs, le procédé selon l'invention est rapide dans la mesure où il consiste à éclairer, mesurer un spectre et réaliser un traitement, notamment informatique, de ce spectre.

Selon un mode de réalisation, ladite bactérie a également une réponse électromagnétique naturelle dans la gamme de longueur d'ondes 750nm-800nm, et:

- l'éclairage de ladite bactérie est également réalisé dans la gamme de longueurs d'onde 750nm-800 nm;
- l'acquisition d'une intensité lumineuse réfléchie par, ou transmise au travers de, la bactérie éclairée est également réalisé dans la gamme 750-800 nm; et
- la détermination du type de Gram la souche bactérienne est réalisée en fonction des intensités lumineuses acquises dans lesdites gammes 415nm-440nm et 750nm-800nm.

En d'autres termes, la bactérie comporte également une signature électromagnétique naturelle dans la gamme 750nm-800nm caractéristique de son Gram. La détection de son Gram peut également être réalisée dans cette gamme. Avantageusement, en combinant les deux gammes de longueurs d'onde, il est obtenu une meilleure précision dans la détermination du type de Gram.

Selon un mode de réalisation, la détermination du type de Gram comprend :

- le calcul d'un facteur de réflectance ou d'absorption de ladite bactérie éclairée en fonction de l'intensité lumineuse acquise ;
- la détermination du type de Gram en fonction du facteur de réflectance ou d'absorption calculé.

En d'autres termes, le facteur de réflectance ou d'absorption reflète plus fidèlement le comportement électromagnétique naturel de la bactérie, car cela corrige le signal mesuré du comportement spectral de l'éclairage et du capteur (e.g. le point blanc, le point noir, la non uniformité du spectre de l'éclairage dans la ou les gammes d'intérêt, etc.) et permet donc une détection plus précise.

En particulier, la détermination du type de Gram comprend :

- le calcul d'une dérivée première en la longueur d'onde du facteur de réflectance ou d'absorption calculé ;
- la détermination du type de Gram en fonction la dérivée première calculée.

En d'autres termes, les inventeurs ont noté que la signature électromagnétique caractéristique du Gram est plus discriminante en dérivant l'intensité lumineuse ou le facteur de réflectance /d'absorption, ce qui permet une détection plus précise.

Selon un mode de réalisation, la détermination du type de Gram est réalisée en fonction de l'intensité lumineuse à une unique longueur d'onde de la gamme 415nm-440nm, en particulier la longueur d'onde 420nm. Plus particulièrement, la détermination du type de Gram comprend :

- la comparaison d'une valeur égale à l'intensité lumineuse à 420nm, ou calculée à partir de celle-ci, à un premier et second seuils prédéterminés séparant la coloration de Gram positive de la coloration de Gram négative, le premier seuil étant supérieur au second seuil ;
- la détermination du Gram négatif si ladite valeur est supérieure au premier seuil et la détermination du Gram positif si ladite valeur est inférieure au second seuil.

En d'autres termes, un bon type de précision dans la détection est obtenu même avec une seule longueur d'onde, en particulier à 420nm. Notamment, un simple seuillage permet d'avoir une précision minimum de 80%. Ceci permet de simplifier les calculs informatiques et/ou l'éclairage et le capteur. La détermination peut être réalisé à partir de l'intensité lumineuse elle-même ou après un prétraitement de celle-ci (e.g. filtrage du bruit, retrait d'un offset, passage à une dérivé première ou seconde, etc.).

Selon un mode de réalisation, la détermination du type de Gram comprend l'application d'une prédiction linéaire prédéterminée reliant l'intensité lumineuse acquise, ou une valeur calculée à partir de celle-ci, au type de Gram, suivie d'un seuillage du résultat de la prédiction linéaire appliquée. Une telle technique permet d'atteindre des typex de précision supérieure à *[à compléter].*

En variante, la détermination du type de Gram comprend l'application d'une classification bi-classe de type SVM à l'intensité lumineuse acquise, ou à une valeur calculée à partir de celle-ci.

Selon un mode de réalisation l'éclairage et l'acquisition sont réalisés directement sur un échantillon comprenant une colonie de le souche bactérienne et un milieu nutritif dans lequel ladite colonie a poussé.

En d'autres termes, l'invention s'applique avantageusement directement sur un échantillon usuellement produit dans le flux de travail microbien, à savoir la pousse de colonie, par exemple sur boites de Pétri, de sorte que l'invention n'implique pas de prévoir une préparation d'échantillon spécifique pour être mise en oeuvre. Par ailleurs, un colonie comporte une quantité très importante de bactéries de la souche. Ainsi mesurer l'intensité réfléchie par, ou transmise au travers, d'une colonie permet de naturellement d'augmenter le degré de fiabilité de la détection, l'intensité étant naturellement moyenner par exemple. On notera cependant que l'invention s'applique à la détection individuelle de bactérie, une étude statistique pouvant être mise en oeuvre lorsque plusieurs bactéries sont observées. De même l'invention s'applique évidement à des milieux différents de ceux usuellement utilisés dans les boites de Pétri (usuellement des géloses), comme par exemple des milieux liquides ayant les bactéries en suspension.

En particulier, la détermination du type de Gram est réalisée en fonction dudit milieu nutritif. En d'autres termes, le milieu nutritif a également sa propre « signature électromagnétique » dans les gammes de longueurs d'onde d'intérêt, et peut donc perturber la détection du type de Gram. En tenant compte du milieu nutritif, soit lors du traitement, soit lors dans le choix de celui utilisé pour la pousse de la colonie, une meilleure précision de détection est obtenue.

En particulier, un substrat opaque à la surface duquel la colonie a poussé, ledit substrat ayant de préférence une réflectance $\rho$ inférieure ou égale à 10%, de préférence inférieure ou égale à 5%. En particulier dans le cadre d'une détection basée sur l'intensité lumineuse réfléchie, une meilleure précision est obtenue.

Selon un mode de réalisation, l'acquisition de l'intensité lumineuse comporte l'acquisition d'une image hyperspectrale ou multispectrale d'une colonie de bactéries de la souche, et l'intensité lumineuse est déterminée en fonction d'au moins un pixel de ladite image correspondant à la colonie. En particulier, l'intensité lumineuse est égale à la moyenne de pixels de ladite image correspondant à la colonie.

En d'autres termes, l'imagerie hyperspectrale ou multispectrale permet d'acquérir de manière simultanée les deux gammes 415nm-440nm et 750nm-800nm à l'aide d'un seul capteur, et permet en outre d'obtenir une moyenne spatiale de chaque longueur d'onde acquise sur plusieurs pixels, ce qui augmente la précision de la détection. Comme cela est connu en soit, le terme « hyperspectral » correspond à l'acquisition d'une gamme de longueurs d'onde dans son ensemble (au pas d'échantillonnage près) alors que le terme « multispectral » renvoie généralement à l'acquisition dans des gammes distinctes.

L'invention a également pour objet un système pour la mise en oeuvre du procédé venant d'être décrit. En particulier, l'invention a pour objet un système de détection du type de Gram d'une souche bactérienne, comprenant :

- un éclairage configuré pour éclairer, dans la gamme de longueurs d'onde 415nm-440nm, au moins une bactérie de la souche;
- un capteur configuré pour acquérir, dans la gamme 415nm-440nm, une intensité lumineuse réfléchie par, ou transmise au travers de, ladite bactérie éclairée; et
- une unité informatique configurée pour déterminer le type de Gram de la souche bactérienne en fonction de l'intensité lumineuse acquise dans la gamme 415nm-440nm.

Selon un mode de réalisation :

- l'éclairage est configuré pour éclairer ladite bactérie dans la gamme de longueurs d'onde 750nm-800 nm ;
- le capteur est configuré pour acquérir, dans la gamme de longueurs d'onde 750nm-800 nm, une intensité lumineuse réfléchie par, ou transmise au travers de, ladite bactérie éclairée; et
- l'unité informatique est configurée pour déterminer le type de Gram de la souche bactérienne en fonction de l'intensité lumineuse acquise dans lesdites gammes 415nm-440nm et 750nm-800nm.

Selon un mode de réalisation, le système est configuré pour éclairer, et acquérir l'image de, un échantillon comprenant une colonie de bactéries de ladite souche et milieu nutritif dans lequel ladite colonie a poussé, en particulier une boite de Pétri.

## BREVE DESCRIPTION DES FIGURES

L'invention sera mieux comprise à la lecture de la description qui va suivre, donnée uniquement à titre d'exemple, et faire en relation avec les dessins annexés, dans lesquels des références identiques désignent des éléments identiques ou analogues, et dans lesquels

- la figure 1 est une vue schématique d'un système de prédiction du type de Gram selon l'invention ;
- la figure 2 est un organigramme d'un procédé de prédiction du type de Gram mis en oeuvre à l'aide du système de la figure 1 ;
- les figures 3A et 3B sont des tracés illustrant des spectres de réflectance de colonies bactériennes ayant poussées sur un milieu transparent ;
- les figures 4A et 4B sont des tracés illustrant des spectres de réflectance de colonies bactériennes ayant poussées sur un milieu opaque ;
- la figure 5 est un tracé illustrant le modèle de prédiction linéaire utilisé pour la prédiction selon une variante de l'invention ;
- la figure 6 est un tracé illustrant la corrélation obtenu par un modèle linéaire mono-canal de prédiction pour les différentes longueurs d'onde d'acquisition ;
- la figure 7 est un tracé illustrant des résultats de prédiction du type de Gram au moyen du modèle linéaire mono-canal de prédiction ;
- la figure 8 illustre une table de différentes variables de décisions pouvant être utilisée pour la prédiction selon l'invention ;
- la figure 9 est un tracé illustrant des résultats de prédiction du type de Gram au moyen d'un modèle linéaire multi-canal de prédiction ;
- la figure 10 est un tracé illustrant les paramètres du modèle multi-canal en fonction des longueurs d'onde d'acquisition ;
- la figure 11 est un tracé illustrant les composantes d'un modèle de prédiction basé sur une classification SVM en fonction des longueurs d'onde d'acquisition ;
- la figure 12 est un tracé illustrant les composantes d'un modèle de prédiction basé sur un apprentissage de type « fused Lasso » en fonction des longueurs d'onde d'acquisition ; et
- les figures 13A et 13B sont des tracés illustrant des spectres de réflectance de colonies bactériennes ayant poussées sur un milieu TSA et MH2.

## DESCRIPTION DETAILLEE DE L'INVENTION

Dans ce qui suit la notation $A_{i,j}$ se rapporte à l'élément de la $i^{ème}$ ligne et de la $j^{ième}$ colonne de la matrice $A$.

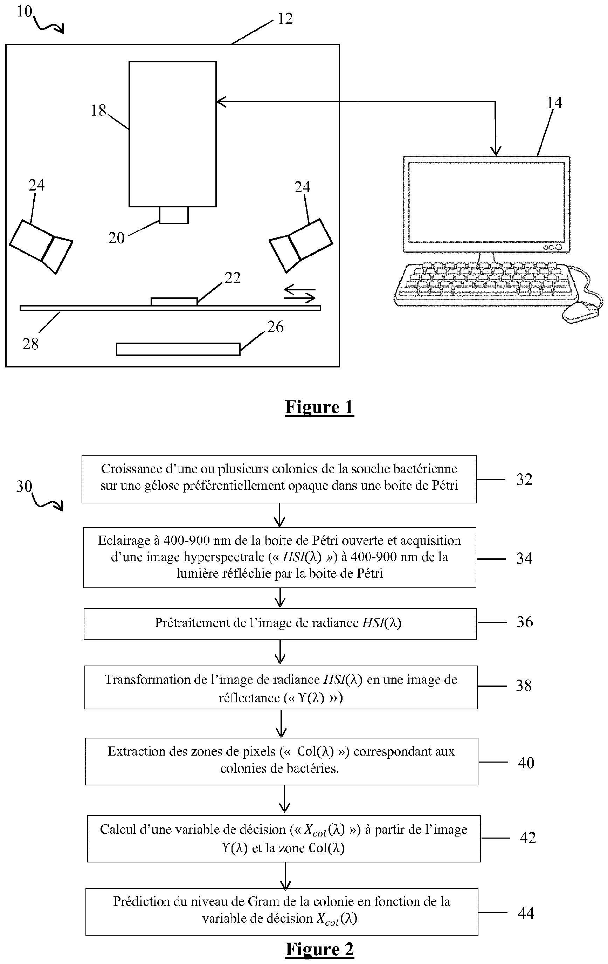

En se référant à la figure 1, un système **10** de détection du type de Gram d'une souche bactérienne selon un premier mode de réalisation comporte :

- un dispositif **12** d'acquisition d'image hyperspectrale ; et
- une unité **14** informatique de traitement de données connectée (e.g. par une liaison filaire ou sans fil), au dispositif **12** pour son pilotage et pour la réception et le traitement des images acquises par le dispositif **12.**

Le dispositif **12**, par exemple un système d'imagerie hyperspectrale de référence « Pika II » de la société Resonon, Montana USA, comporte :

- une caméra dite « hyperspectrale » **18**, constituée d'un capteur numérique comprenant un réseau de capteurs élémentaires, par exemple un capteur numérique de type CCD ou CMOS, sensible dans la gamme de longueurs d'onde $[\lambda_{min}; \lambda_{max}] = [400; 900]$ nanomètres, et d'un élément dispersif de lumière ou d'un spectrographe pour sélectionner une longueur d'onde à acquérir par le capteur ;
- un objectif **20** pour focaliser sur le capteur numérique de la caméra **18**, l'image optique d'une boite de Pétri **22**, dont on cherche à acquérir une image hyperspectrale ;
- un éclairage avant **24**, par exemple constitué d'une ou plusieurs lampes allogènes, e.g. 2 ou 4 lampes, apte à émettre de la lumière dans la gamme $[\lambda_{min}; \lambda_{max}]$ et pour réaliser un éclairage avant uniforme de la boite de Pétri **22**. Par exemple, les éclairages sont des lampes à lumière blanche ;
- un éclairage arrière **26**, par exemple constitué d'une matrice de LED à lumière blanche, pour réaliser un éclairage arrière uniforme de la boite de Pétri **22** dans la gamme $[\lambda_{min}; \lambda max]$ ; et

- un chariot **28** sur lequel repose boite de Pétri **22** et permettant à cette dernière de défiler devant l'objectif **20** afin d'obtenir une image entière de la boite **22** par balayage.

Le dispositif **12** est par exemple configuré pour acquérir l'image d'une région de 90 millimètres par 90 millimètres avec un pas d'échantillonnage de 160 micromètres (résolution spatiale estimée à 300 micromètres) et avec une résolution spectrale de 1,7 nanomètre sur la gamme $[\lambda_{min}; \lambda_{max}]$.

Le dispositif **12** produit ainsi une image numérique *HSI* de la lumière réfléchie par la boite de Pétri, ayant N lignes et M colonnes, la boite de Pétri **22** étant préférablement ouverte (i.e. sans son couvercle):

$$HSI(\lambda) = \begin{pmatrix} Rad_{1,1}(\lambda) & ... & Rad_{1,j}(\lambda) & ... & Rad_{1,M}(\lambda) \\ \vdots & \ddots & \vdots & \vdots & \vdots \\ Rad_{i,1}(\lambda) & ... & Rad_{i,j}(\lambda) & ... & Rad_{i,M}(\lambda) \\ \vdots & \vdots & \vdots & \ddots & \vdots \\ Rad_{N,1}(\lambda) & ... & Rad_{N,j}(\lambda) & ... & Rad_{N,M}(\lambda) \end{pmatrix} \qquad (1)$$

La radiance d'un pixel, communément appelée « intensité lumineuse », correspond ici à la quantité de lumière incidente sur la surface du site sensible élémentaire correspondant du capteur de la caméra **18** pendant la durée d'exposition, comme cela est connu en soi du domaine de la photographie numérique par exemple.

Chaque pixel $Rad_{i,j}(\lambda)$ se compose d'un spectre numérique de la radiance de la boite **22** correspondant au pixel à différentes longueurs d'onde $[\lambda_{min}; \lambda_{max}]$, le spectre numérique s'exprimant selon la relation :

$$\forall(i,j)\epsilon[1,N] \times [1,M]: Rad_{i,j}(\lambda) = \begin{pmatrix} Rad_{i,j}(\lambda_{min}) \\ Rad_{i,j}(\lambda_{min} + \Delta\lambda) \\ Rad_{i,j}(\lambda_{min} + 2 \times \Delta\lambda) \\ \vdots \\ Rad_{i,j}(\lambda_{min} + p \times \Delta\lambda) \\ \vdots \\ Rad_{i,j}(\lambda_{max}) \end{pmatrix} \qquad (2)$$

où $\Delta\lambda$ est la résolution spectrale et *p* est un entier positif appartenant à $\left[0, P = \frac{\lambda_{max} - \lambda_{min}}{\Delta\lambda}\right]$. Les longueurs d'onde d'acquisition $\lambda_{min} + p \times \Delta\lambda$ sont usuellement désignées sous le termes de « canaux ».

L'unité **14** de traitement de données est par exemple un ordinateur personnel, une tablette, un smartphone, un serveur, un supercalculateur, ou plus généralement tout système à base de microprocesseur(s), notamment de type DSP (« digital signal processor »), à base de circuits de type FPGA, à base de circuits mixant ces types de technologie, etc., configuré pour mettre en oeuvre un traitement des images *HSI* produites par le dispositif d'acquisition **12.** L'unité **14** est notamment pourvue de l'ensemble des mémoires (RAM, ROM, cache, mémoire de masse,...) pour la mémorisation des images produites par le dispositif **12**, d'instructions informatiques pour la mise en oeuvre du procédé selon l'invention, de paramètres utiles à cette mise en oeuvre et pour la mémorisation des résultats des calculs intermédiaires et finaux, notamment le type de Gram déterminé. L'unité **14** comporte optionnellement un écran d'affichage pour la visualisation du résultat final de la détermination du type de Gram. Bien qu'une seule unité de traitement soit décrite, l'invention s'applique évidemment à un traitement réalisé par plusieurs unités de traitement (e.g. une unité embarquée dans la caméra **18** pour mettre en oeuvre un prétraitement de images *HSI* et une unité externe au dispositif **12** pour la mise en oeuvre du reste du traitement). Par ailleurs, le système peut être complété par une interface permettant d'entrer dans l'unité **14** des données relatives à l'échantillon, notamment le type de milieu de culture utilisé lorsque la prédiction dépend du milieu, par exemple au moyen d'un clavier/souris et d'un menu déroulant à disposition de l'opérateur, un lecteur de code barre/QR code lisant un code barre/QR code présent sur la boite de Pétri et comprenant des informations sur le milieu, etc.

Un procédé **30** de détermination du type de Gram d'une souche bactérienne au moyen du système venant d'être décrit est à présent détaillé en relation avec l'organigramme de la figure 2.

Dans une première étape **32** du procédé, au moins une colonie de la souche bactérienne est cultivée à la surface d'un milieu nutritif, ou « de culture », déposé dans une boîte de Pétri. Le milieu nutritif, a pour but principal de faire pousser ladite colonie, et optionnellement de renforcer la précision de la détection du type de Gram en limitant les perturbations lumineuses. De préférence concernant une détection du type de Gram en fonction de l'intensité lumineuse réfléchie, le milieu nutritif est opaque, ce qui augmente le degré de précision de la détection. Notamment, le milieu opaque a un facteur de réflectance $\rho$ inférieur ou égal à 10%, et de préférence inférieur ou égal à 5%, et de manière encore plus préférentielle inférieur ou égal à 1%. Par exemple, le milieu de culture une gélose dite « CPSO » (gélose « CPS » comprenant du SiO$_2$ pour opacifier le milieu), une gélose dite « columbia » (ou gélose « CNA »), une gélose Columbia avec 5% de sang de mouton (ou gélose dite « COS »), une gélose de Man, Rogosa, Sharpe (gélose dite « MRSM »), une gélose chocolat (gélose dite « PVX »), etc.

Ce type de pousse de colonies étant classique, il ne sera pas décrit plus en détail par la suite. Il peut avantageusement être mis en oeuvre manuellement par un opérateur ou automatiquement à l'aide d'un automate d'ensemencement d'une manière connue en soi. De manière avantageuse, la préparation est réalisée de sorte que les colonies, sur la base desquels la prédiction du type de Gram est réalisée, sont distancées les unes des autres et de sorte que la surface d'une colonie corresponde à une pluralité de pixels dans l'image acquise par le dispositif 12. Ceci permet notamment de faciliter leur identification ultérieure dans l'image acquise.

Une fois la pousse des colonies terminée, la boite de Pétri est ouverte, disposée sur le chariot 28, les éclairages 24 et 26 sont allumés et au moins une image hyperspectrale *HSI* de la boite de Pétri est acquise, en 34, à l'aide du dispositif d'acquisition 12 et mémorisée dans l'unité de traitement 14, qui met en oeuvre un traitement informatique pour déterminer le type de Gram de la souche à partir des images acquises.

L'unité 14 débute optionnellement, en 36, par un prétraitement du bruit, consistant en l'un des traitements suivants ou une combinaison quelconque de ces traitements :

a. une correction du bruit du capteur de la caméra 18, notamment son offset, son bruit spatial, etc., d'une manière connu en soi ;

b. un traitement des réflexions parasites, notamment spéculaire formant des « surbrillances » dans l'image *HSI.* Par exemple, un seuillage mis en oeuvre pour éliminer les pixels ayant des valeurs supérieures à un seuil prédéterminé, e.g. supérieures ou égales aux deux tiers de la valeur maximale que peuvent prendre les pixels (i.e. supérieures ou égales à 170 dans le cas de pixels codés sur 8 bits entre 0 et 255) ;

c. une traitement ratiométrique permettant d'atténuer les variations dans les images provoquées par des fluctuations extérieures telles que de variations de l'illumination, en divisant l'image *HSI* par une intensité lumineuse réfléchie à une longueur d'onde qui est invariante avec le type de bactérie et le type de gélose utilisé ;

d. si plusieurs images *HSI* ont été acquises, la recherche et l'élimination de valeurs de pixels aberrantes et/ou la moyenne des images acquises.

Le traitement se poursuit, en 38, par la transformation de l'image *HSI* prétraitée, qui mémorise des valeurs de radiance à différentes longueurs d'onde, en une image hyperspectrale de réflectance afin d'extraire le signal généré par la boite de Pétri seule. Ceci permet notamment de filtrer les fluctuations du spectre d'émission des sources d'éclairage 24, 26. Par exemple, une correction du type « *flat field correction* » (FFC) est mise en oeuvre pour obtenir la réflectance, ce qui présente en outre l'avantage de corriger les dispersions de réponse du capteur de pixel à pixel (dispersion du courant d'obscurité, dispersion du gain, etc.). Cette transformation est par exemple une correction selon les relations :

$$\forall (i,j) \epsilon [1,N] \times [1,M], \forall p \in [0,P]:$$

$$\Upsilon_{i,j}(\lambda_{min} + p \times \Delta\lambda)$$
$$= \frac{Rad_{i,j}(\lambda_{min} + p \times \Delta\lambda) - B_{i,j}(\lambda_{min} + p \times \Delta\lambda)}{W_{i,j}(\lambda_{min} + p \times \Delta\lambda) - B_{i,j}(\lambda_{min} + p \times \Delta\lambda)} \times m(\lambda_{min} + p \times \Delta\lambda) \tag{3}$$

où $\Upsilon(\lambda)$ est une image en réflectance, $W$ est une image hyperspectrale mémorisée dans l'unité 14 d'un objet neutre de réflectance élevée et illuminé par les éclairages 24, 26, par exemple une feuille de réflectance uniforme supérieure à 90% (e.g. une feuille dite « blanche » ou à une charte de gris inférieur à 10%), et $B$ est une image hyperspectrale mémorisée dans l'unité 14 d'un objet neutre de réflectance faible, par exemple l'image d'un capuchon noir obturant l'objectif 20 et $m(\lambda_{min} + p \times \Delta\lambda = 1$ ou égal à la moyenne de la matrice $W(\lambda_{min} + p \times \Delta\lambda)$ - $B(\lambda_{min} + p \times \Delta\lambda)$.

L'unité 14 met en oeuvre en 40, suite à l'étape 38 ou en parallèle des étapes précédentes, un algorithme d'identification des colonies de bactéries, e.g. à partir de l'image *HSI*($\lambda$) ou $\Upsilon(\lambda)$. Tout algorithme de reconnaissance de forme et d'objet classique peut être mis en oeuvre pour extraire une zone de l'image, nommée « Col($\lambda$) », correspondant à une colonie. En variante, cette sélection est faite manuellement par un opérateur qui sélectionne cette zone en s'aidant de l'écran d'affichage et d'un mécanisme de pointage du type souris par exemple. A titre d'exemple, la zone Col($\lambda$) consiste en une liste des coordonnées de pixel appartenant à la colonie. Les zones de pixels sélectionnées sont mémorisées par l'unité **14**.

Le procédé se poursuit, en **42**, par le calcul d'une variable de décision $X_{col}(\lambda)$ en fonction des pixels de l'image $\Upsilon(\lambda)$ compris dans la zone Col($\lambda$), cette variable étant celle sur laquelle des règles de prédiction du type de Gram s'appliquent. Dans une variante, la variable de décision $X_{col}(\lambda)$ est basée uniquement sur l'image $\Upsilon(\lambda)$. Dans d'autres variantes, l'image $\Upsilon(\lambda)$ est complétée ou remplacée par un ou plusieurs autres types de grandeurs calculées en fonction de l'image de réflectance $\Upsilon(\lambda)$, comme cela sera décrit plus en détail par la suite. A cet effet, un traitement de l'image en réflectance $\Upsilon(\lambda)$ est donc, optionnellement, mis en oeuvre. En particulier, l'unité **14** calcule l'une et/ou l'autre des grandeurs suivantes :

> i. une image en absorbance A($\lambda$), par exemple au moyen la transformée de Beer-Lamber (relation (4)) ou de la transformé de Kubelka-Munk (relation (5)). Sans être lié par la théorie, les inventeurs sont d'avis que les bactéries de Gram négatif diffèrent des bactéries de Gram positif par une teneur plus importante en cytochromes qui sont absorbants dans la gamme [415-440]nm, ce que permet de caractériser la transformation en image d'absorbance.

$$\forall(i,j)\epsilon[1,N]\times[1,M], \forall p \in [0,P]:$$

$$A_{i,j}(\lambda_{min} + p \times \Delta\lambda) = log\left(\frac{1}{\Upsilon_{i,j}(\lambda_{min} + p \times \Delta\lambda)}\right) \qquad (4)$$

$$A_{i,j}(\lambda_{min} + p \times \Delta\lambda) = \frac{\left(1 - A_{i,j}(\lambda_{min} + p \times \Delta\lambda)\right)^2}{2 \times A_{i,j}(\lambda_{min} + p \times \Delta\lambda)} \qquad (5)$$

> ii. une image en réflectance $\Upsilon(\lambda)$ normalisée ou une image en absorbance A($\lambda$) normalisée afin de réduire les variabilités dues aux effets optiques. L'unité met par exemple en oeuvre une normalisation par zone (connue sous le nom de « Area normalization »), une normalisation par vecteur unitaire (connue sous le nom de « Unit vector normalization »), une normalisation moyenne (connue sous le nom de « Mean normalization »), une normalisation par maximum (connue sous le nom de « Maximum normalization »), une normalisation par intervalle (connue sous le nom de « Range normalization ») ou une normalisation par pic (connue sous le nom de « Peak normalization »). Ces normalisations sont décrites par exemple dans le document de K. Varmuza et P. Filzmoser, "Introduction to Multivariate Statistical Analysis in Chemometrics", CRC Press, 2009 et le document de A. Rinnan, F. van den Berg et S. Engelsen, "Review of the most common pre-processing techniques for near-infrared spectra", Trends in Analytical Chemistry, vol. 28, n° 10, 2009.
>
> iii. la dérivée première $\frac{\partial}{\partial\lambda}$ ou seconde $\frac{\partial^2}{\partial\lambda^2}$ en la longueur d'onde de l'image en réflectance $\Upsilon(\lambda)$, normalisée ou non, ou de l'image en absorbance A($\lambda$), normalisée ou non, afin d'accentuer les changements de comportement dans le spectre et réduire les lignes de base. De préférence, un algorithme de filtrage local polynomial est mis en oeuvre pour réduire la propagation d'erreur, avantageusement un algorithme tel que décrit dans le document de A. Savitzky et M.J.E. Golay « Smoothing and differentialtion of data by simplified least squares procédures », Anal. Chem., vol.36, pp.1627-1639, 1964.

Le type de Gram de chaque colonie est ensuite prédit en **44** par l'unité **14** en fonction de la variable $X_{col}(\lambda)$ en appliquant des règles de décision prédéfinies, dont des variantes sont décrites ci-après. Le type de Gram prédit est mémorisé dans l'unité **44** et/ou affiché sur un écran. Cette prédiction est par exemple délivrée à un autre instrument d'analyse microbienne pour une étape ultérieure d'identification/caractérisation de la colonie.

A. APPROCHE MONO-CANAL

Dans une première variante, une approche mono-variable et mono-canal est mise en oeuvre par l'unité **14**. Selon cette approche, la variable de décision $X_{col}(\lambda)$ se résume à une unique valeur prise à une longueur d'onde

prédéfinie $\lambda_c$ dans la gamme de longueurs d'onde 415nm-440nm, e.g. la réflectance de la colonie (normalisée ou non), ou l'absorbance de la colonie (normalisée ou non), ou la dérivée première ou la dérivé seconde de celle-ci.

Dans une première variante basée sur la réflectance, l'unité **14** calcule, en **42**, un spectre en réflectance de la colonie, e.g. un spectre moyen sur les pixels de la colonie selon la relation :

$$\forall p \in [0, P]: \Upsilon_{col}(\lambda_{min} + p \times \Delta\lambda) = \frac{1}{N_{col}} \times \sum_{(i,j) \in \mathrm{Col}(\lambda)} \Upsilon_{i,j}(\lambda_{min} + p \times \Delta\lambda) \qquad (6)$$

où $N_{col}$ est le nombre de pixels de la zone Col($\lambda$). La variable de décision $X_{col}(\lambda)$ est alors égale à $\Upsilon_{col}(\lambda_c)$. L'unité **14** calcule également une erreur de mesure de la colonie à la longueur d'onde $\lambda_c$, en particulier l'écart type, noté « *SD* », de l'ensemble des valeurs $\Upsilon_{i,j}(\lambda_c)$ des pixels appartenant à la zone de pixels Col($\lambda$).

En **44**, l'unité **44** applique alors les règles de comparaison suivantes à la valeur du spectre $\Upsilon col(\lambda_c)$ :

$$\mathrm{si}\ \Upsilon_{col}(\lambda_c) < \Upsilon_{GN_{max}}(\lambda_c) + SD \ \mathrm{alors\ la\ colonie\ est\ de\ Gram\ négatif} \qquad (7)$$

$$\mathrm{si}\ \Upsilon_{col}(\lambda_c) > \Upsilon_{GP_{min}}(\lambda_c) - SD \ \mathrm{alors\ la\ colonie\ est\ de\ Gram\ positif} \qquad (8)$$

$$\mathrm{si}\ \Upsilon_{col}(\lambda_c) \in \left[\Upsilon_{GP_{min}}(\lambda_c) - SD; \Upsilon_{GN_{max}}(\lambda_c) + SD\right] \ \mathrm{alors\ le\ Gram\ est\ indéterminé} \qquad (9)$$

où $\Upsilon_{GN_{max}}(\lambda_c)$ et $\Upsilon_{GP_{min}}(\lambda_c)$ sont deux seuils prédéfinis, dépendant préférentiellement du milieu de culture et mémorisés dans l'unité **14**. En variante, l'erreur de mesure *SD* peut être choisie nulle.

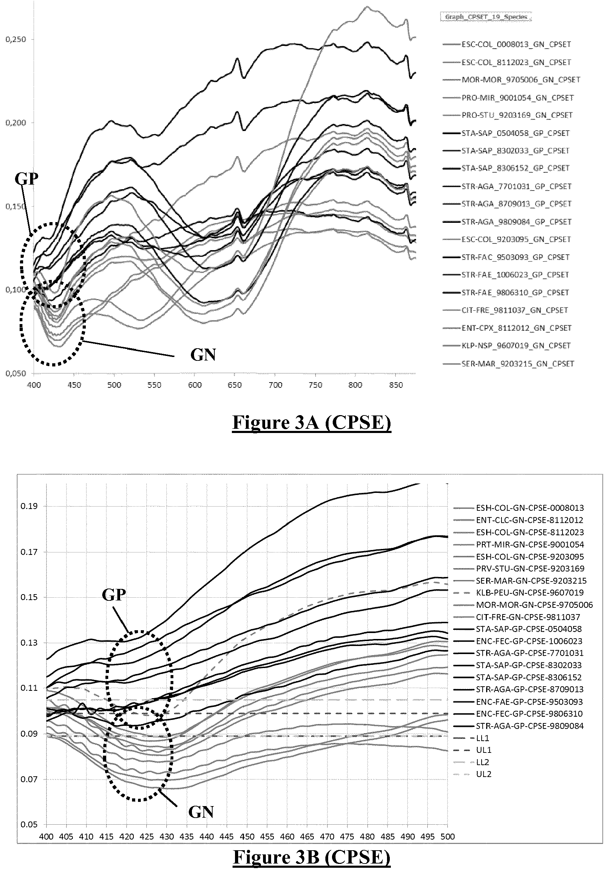

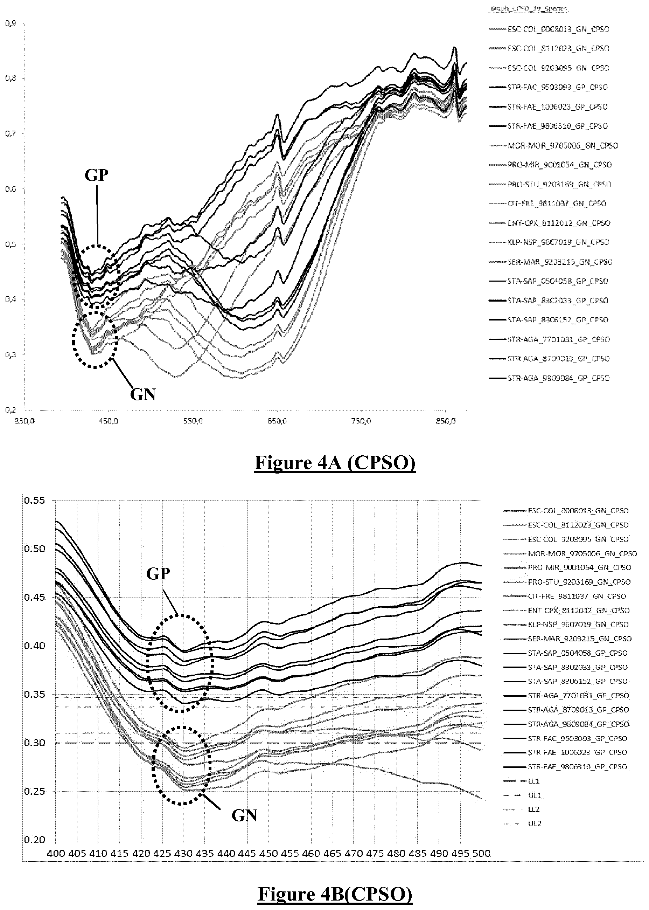

La figure 3A illustre des spectres moyens $\Upsilon_{col}(\lambda)$ de colonies ayant poussées sur un milieu transparent (ici du CPSE), à savoir 10 souches de Gram négatif (« GN ») (*Escherichia coli* (3 souches), *Enterobacter cloacae, Proteus mirabilis, Providencia stuartii, Serratia marcescens, K. pneumoniae, Morganella morganii, Citrobacter freundii*) et 10 souches de Gram positif (« GP ») (*Staphylococcus saprophyticus, Enterococcus faecalis* (2 souches), *Streptococcus agalactiae, Staphylococcus saprophyticus* (2 souches), *Streptococcus agalactiae, Enterococcus faecium, Streptococcus agalactiae*). La figure 3B est une vue plus en détail de la gamme 415-440nm. Les figures 4A et 4B illustrent ces mêmes spectres mais pour des souches ayant poussé sur un milieu opaque (ici du CPSO , différent du CPSE en ce qu'il contient des particule de SiO$_2$). Comme on peut le constater sur ces exemples, au moins dans la gamme 415-440 nm, les deux types de Gram se distinguent l'un de l'autre, la différence étant même très marquée pour le milieu opaque. En particulier, dans la gamme 415-440 nm, on observe que les spectres des bactéries de Gram négatif est inférieur aux spectres des bactéries de Gram positif.

La première variante a été testée sur 30 souches bactériennes, cultivées pour chaque milieu de culture, parmi les plus fréquemment impliquées dans les infections cliniques, à savoir (nombre de souches par espèce entre parenthèse):

- pour le Gram négatif : *Escherichia coli* (1), *K.pneumoniae* (1), *Klebsiella oxytoca (1), Proteus mirabilis* (1), *Morganella morganii* (1)*, Stenotrophomonas maltophilia* (1), *Acinetobacter baumanii* (1), *Enterobacter cloacae* (1), *Serratia marcescens* (1), *Citrobacter koseri* (1), *Enterobacter aerogenes* (1), *Moraxella catarrhalis (1)*, *Hafnia alvei* (1), *Pseudomonas aeruginosa* (1), *Haemophilus influenzae* (1) ;
- pour le Gram positif : *Corynebacterium jeikeium* (1), *Corynebacterium amycolatum* (1), *Enterococcus faecalis* (1), *Enterococcus faecium*(1), *Staphylococcus aureus*(1), *Staphylococcus epidermidis* (1), *Staphylococcus haemolyticus* (1), *Staphylococcus hominis* (1), *Streptococcus pneumoniae* (1), *Streptococcus agalactiae* (1), *Streptococcus pyogenes* (1), *Streptococcus anginosus* (1), *Streptococcus viridans* (1).

Pour chaque canal $\lambda$ de la gamme 415-440 nm, le minimum $\Upsilon_{GP_{min}}(\lambda)$ des spectres à la valeur $\lambda$ des souches de Gram positif est déterminé. De même, le maximum $\Upsilon_{GN_{max}}(\lambda)$ des spectres à la valeur $\lambda$ des souches de Gram négatif est déterminé. Le canal $\lambda_c$ retenu pour le milieu de culture est alors celui qui maximise l'intervalle $[\Upsilon_{GP_{min}}(\lambda_c); \Upsilon_{GN_{max}}(\lambda_c)]$, et les seuils prédéfinis retenus pour le milieu sont respectivement les valeurs $\Upsilon_{GP_{min}}(\lambda_c)$ et $\Upsilon_{GN_{max}}(\lambda_c)$. Pour le milieu CPSE, le type de Gram de 19 souches d'espèces identiques à celles listées ci-dessus, dont 10 à Gram positive et 9 à Gram positive, a été prédit avec un taux de succès proche de 90%. Pour le milieu CPSO, le succès sur ces mêmes souches est de 100%.

Dans une deuxième variante basée sur la dérivée première de la réflectance, l'unité **14** calcule, en **42**, un spectre en réflectance de la colonie, e.g. un spectre moyen $\Upsilon_{col}(\lambda)$ sur les pixels de la colonie tel que décrit ci-dessus, puis calcule la dérivée première $\frac{\partial \Upsilon_{col}}{\partial \lambda}(\lambda_c)$. du spectre moyen. La variable de décision $X_{col}(\lambda)$ est alors égale à $\frac{\partial \Upsilon_{col}}{\partial \lambda}(\lambda_c)$. En **44**, l'unité **14** applique un modèle de prédiction à ladite variable, par exemple un modèle linéaire discriminant selon les relations :

$$Gram = b + a \times \frac{\partial \Upsilon_{col}}{\partial \lambda}(\lambda_c) \qquad (10)$$

$$Si\ Gram < 0,5\ \text{alors la colonie est de Gram négatif} \qquad (11)$$

$$Si\ Gram > 0,5\ \text{alors la colonie est de Gram positif} \qquad (12)$$

où *Gram* est un score, les paramètres *a* et *b* sont des coefficients prédéfinis mémorisés dans l'unité **14**, et dépendant préférentiellement du milieu de culture.

La seconde variante a été testée sur 52 souches, 28 de Gram négatif et 24 de Gram positifs, appartenant à 19 espèces différentes parmi les plus fréquemment impliquées dans les infections cliniques, à savoir (nombre de souches par espèce entre parenthèse) :

- pour le Gram négatif : *Escherichia coli* (9), *Klebsiella pneumoniae* (3), *Enterobacter cloacae* (1), *Serratia Marcescens* (2), *Citrobacter freundii* (2), *Proteus mirabilis* (2), *Providencia stuartii* (1), *Morganella morganii* (2) ;
- pour le Gram positif : *Staphylococcus saprophyticus* (9), *Streptococcus agalactiae* (7), *Enterococcus faecium* (3), *Enterococcus faecalis* (3), *Staphylococcus aureus* (1), *Candida albicans* (1).

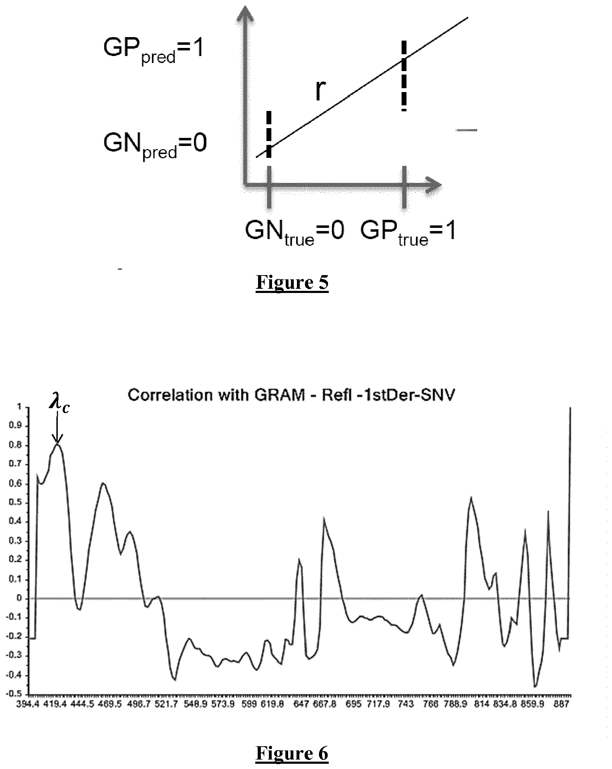

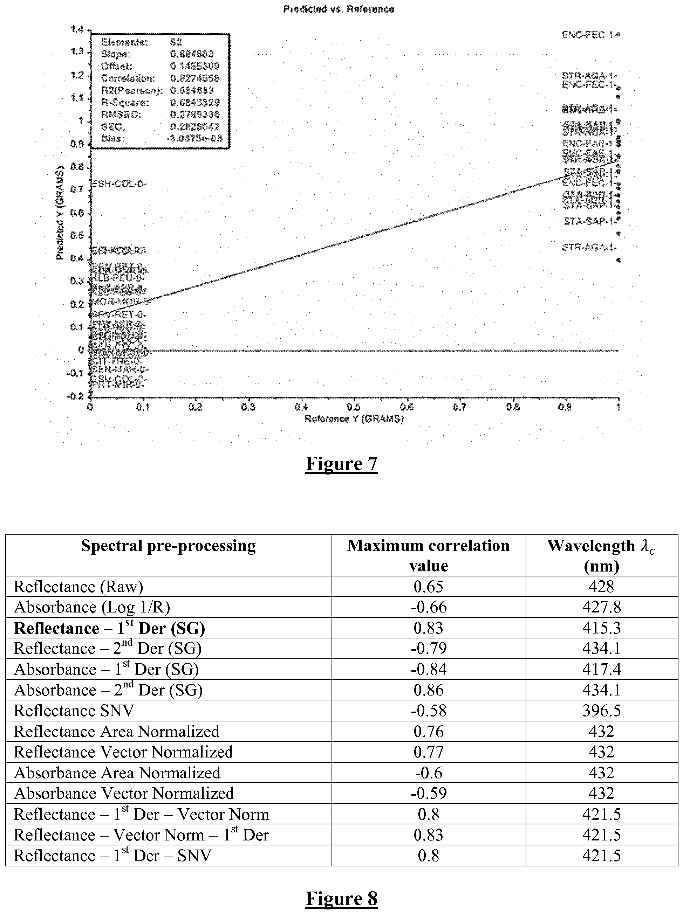

La figure 5 illustre le modèle d'apprentissage de la relation (10), le Gram négatif observé se voyant assigner la valeur 0 et le Gram positif observé se voyant assigner la valeur 1. Une régression linéaire entre les Gram observés et prédits est mise en oeuvre pour la détermination des paramètres *a* et *b* d'une manière connue en soi. Le modèle a été appris pour chaque longueur d'onde de la gamme 400-900nm, et la figure 6 illustre le coefficient de corrélation en fonction de la longueur d'onde pour un milieu de culture transparent (ici CPSE). Le maximum de ce corrélogramme correspond à la longueur d'onde $\lambda_c$ et on observe ainsi que le caractère discriminant de la gamme 415-440nm est automatiquement retrouvée par régression linéaire, $\lambda_c$ étant en effet égal à 415,3 nanomètres. La figure 7 illustre le Gram prédit en fonction du Gram connu des souches décrites ci-dessus pour le milieu CPSE, le taux de succès étant supérieur à 80%. Le taux de succès pour un milieu opaque est par ailleurs de 100%. Plusieurs variables de décision ont été testée, la table de la figure 8 listant celles-ci. Il apparait que les dérivées première et seconde de la réflectance non normalisée sont les variables menant à un taux de succès le plus élevé, la dérivée première étant cependant préférée selon le principe de parcimonie.

B. APPROCHE MULTI-CANAL

Selon cette approche, plusieurs canaux de la gamme de longueurs d'onde 415-440 nm sont utilisés, avantageusement et optionnellement en combinaison avec des canaux de la gamme 750-800nm. La variable de décision est $X_{col}(\lambda)$ est par exemple le spectre moyen de réflectance (normalisé ou non), d'absorbance (normalisé ou non), la dérivé première ou la dérivée seconde sur l'ensemble de la gamme 400-900nm.

Selon une première variante, et pour les raisons évoquées ci-dessus, la dérivée première de la réflectance non normalisée est utilisée. L'unité **14** calcule donc, en **42**, la dérivée première $\frac{\partial \Upsilon_{col}(\lambda)}{\partial \lambda}$ du spectre moyen $\Upsilon_{col}(\lambda)$ sur la gamme 400-900nm, comme décrit précédemment, cette dérivée constituant la variable de décision $X_{col}(\lambda)$. En **44**, l'unité **14** applique un modèle de prédiction à ladite variable, par exemple un modèle linéaire selon les relations :

$$Gram = b + \sum_{p \in [0,P]} a_p \times \frac{\partial \Upsilon_{col}(\lambda_{min} + p \times \Delta\lambda)}{\partial \lambda} \qquad (13)$$

$$\text{Si } Gram < 0,5 \text{ alors la colonie est de Gram négatif} \qquad (14)$$

$$\text{Si } Gram > 0,5 \text{ alors la colonie est de Gram positif} \qquad (15)$$

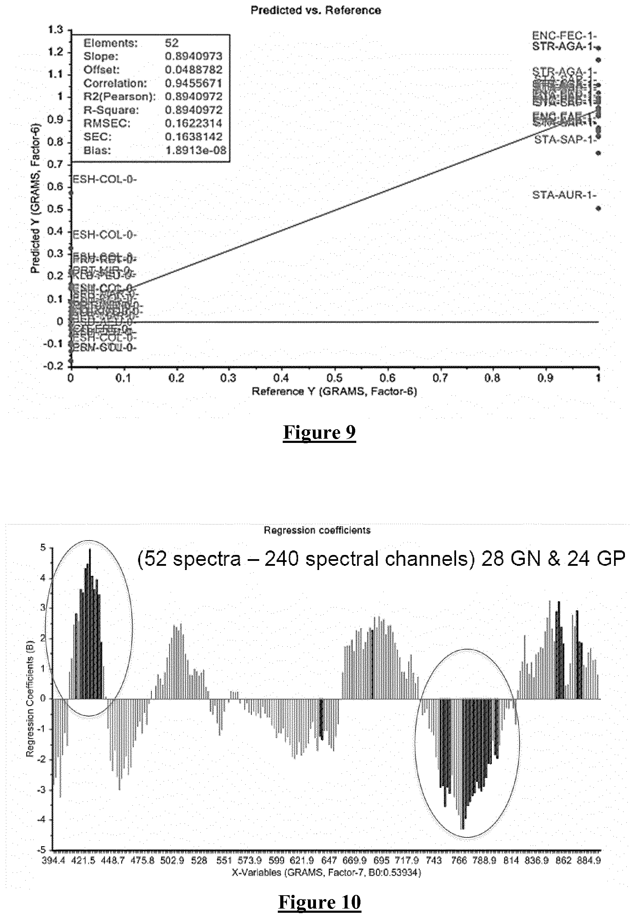

où $Gram$ est un score, les paramètres $a_p$ et $b$ sont des coefficients prédéfinis mémorisés dans l'unité **14**, et dépendant préférentiellement du milieu de culture. Le modèle de prédiction de la relation (13) est appris de manière similaire à celle décrite précédemment, par régression linéaire. La figure 9 illustre le Gram prédit en fonction du Gram observé pour le milieu CPSE. Pour ce dernier, le taux de corrélation est de 95%. La figure 10 illustrent les coefficients $a_p$. On note ici que le modèle de prédiction utilise deux gammes principales de longueurs d'onde dans le modèle de prédiction, à savoir la gamme 415-440nm, et plus particulièrement la gamme 421-436nm, et la gamme 750-800nm. On observe ainsi que la gamme 750-800nm comporte également des informations spectrales discriminantes concernant le type de Gram des bactéries.

Selon une seconde variante, la variable de décision est multi-canal et également multi-pixel, l'information spectrale de chaque pixel de la colonie étant utilisée, et non pas moyennée comme décrit précédemment. Par opposition au terme « moyen », l'information spectrale associée à un pixel est désignée sous le terme « d'individuelle ». En particulier, la variable de décision est $X_{col}(\lambda)$ est constitué de l'ensemble des spectres individuels de réflectance (normalisé ou non), d'absorbance (normalisé ou non), de leur dérivées premières ou de leurs dérivées secondes, des pixels de la zone Col($\lambda$).

Par exemple, en considérant les spectres de réflectance $\{\Upsilon_{i,j}(\lambda)\}_{(i,j) \in Col(\lambda)}$ normalisé (e.g. en divisant chaque spectre individuel par sa norme euclidienne) sur la gamme 400-900nm, l'unité **14** met en oeuvre en **44** une règle de prédiction basée sur une classification à deux classes (i.e. le Gram positif, assigné par exemple aux valeurs positives, et le Gram négatif, assigné par exemple aux valeurs négatives) selon les relations :

$$Gram = X_{col}(\lambda).\hat{\beta} \qquad (16)$$

$$\text{Si } Npos(Gram) > Nneg(Gram), \text{ alors la colonie est de Gram positif} \qquad (17)$$

$$\text{Si } Npos(Gram) < Nneg(Gram), \text{ alors la colonie est de Gram négatif} \qquad (18)$$

où $Gram$ est un vecteur de dimension égale au nombre $P$ de canaux dans la gamme 400-900nm, $X_{col}(\lambda)$ est ici une matrice dont les lignes sont respectivement égales aux spectres individuels de réflectance $\{\Upsilon_{i,j}(\lambda)\}_{(i,j) \in Col(\lambda)}$ normalisés, $\hat{\beta}$ est un vecteur colonne prédéfini de dimension égale à $P$, $Npos(Gram)$ est le nombre de composantes du vecteur $Gram$ qui sont positives et $Nneg(Gram)$ est le nombre de composantes du vecteur $Gram$ qui sont négatives. Les paramètres $\hat{\beta}$ et $\beta_0$, mémorisés dans l'unité **14**, sont préférentiellement dépendant du milieu de culture. La prédiction, basé sur un vote majoritaire, peut en variante être réalisée sur la moyenne du vecteur $\hat{\beta}$, le Gram négatif étant prédit si cette moyenne est négative et le Gram positif étant prédit si cette moyenne est positive.

Dans un première exemple, le modèle de prédiction de la relation (14) est appris sur les pixels des 52 souches précédemment décrites en résolvant un problème d'optimisation de type SVM (pour « Support Vector Machine ») selon les relations suivante :

$$\hat{\beta} = \arg \min_{\beta, \xi_m} \left( \frac{1}{2} \|\beta\|^2 + C \sum_{m=1}^{M} \xi_m \right) \qquad (19)$$

sous les contraintes :

$$\forall m \in [1, M] : \xi_m \geq 0 \qquad (21)$$

$$\forall m \in [1, M] : q_m ( \Upsilon_m(\lambda).\beta) \geq 1 - \xi_m \qquad (22)$$

$$\|\beta\|^2 + C \sum_{m=1}^{M} \xi_m$$

où $N$ est le nombre de spectres individuels de réflectance normalisés, notés $\Upsilon_m(\lambda)$, utilisés pour l'apprentissage, numérotés de 1 à M, $q_m \in \{-1, 1\}$ avec $q_m = 1$ si le $m^{\text{ième}}$ spectre est associé à une bactérie de Gram positif, et $q_m = -1$ si le $m^{\text{ième}}$ spectre est associé à un bactérie de Gram négatif, et $C$ est un scalaire prédéfini.

Le modèle a été validé sur 10 souches de Gram négatif (*Escherichia coli* (3 souches), *Enterobacter cloacae, Proteus mirabilis, Providencia stuartii, Serratia marcescens, K. pneumoniae, Morganella morganii, Citrobacter freundii*) et 10 souches de Gram positif (*Staphylococcus saprophyticus, Enterococcus faecalis* (2 souches), *Streptococcus agalactiae, Staphylococcus saprophyticus* (2 souches), *Streptococcus agalactiae, Enterococcus faecium, Streptococcus agalactiae*). Pour un milieu opaque (e.g. CPSO), le taux de succès de 100%. Pour un milieu transparent (e.g. CPSE), le taux de succès proche de 94% pour les bactéries de Gram négatif et de près de 98% pour les bactéries de Gram positif. Comme on peut le noter à la figure 10, qui illustre les composantes du vecteur $\hat{\beta}$ en fonction des canaux, la prédiction basée sur la classification SVM ne laisse pas apparaître une gamme particulière de longueurs d'onde.

Dans un deuxième exemple, le modèle de prédiction de la relation (14), avec a été appris sur les pixels des 52 souches précédemment décrites en résolvant un problème d'optimisation de type « Fused Lasso » selon la relation suivante:

$$\hat{\beta} = \arg \min_{\beta} \left( \|q_m - \Upsilon_m(\lambda)\|_2^2 + \mu_1 \sum_{p=1}^{P} |\beta_p| + \mu_2 \sum_{p=1}^{P-1} |\beta_{p+1} - \beta_p| \right) \qquad (23)$$

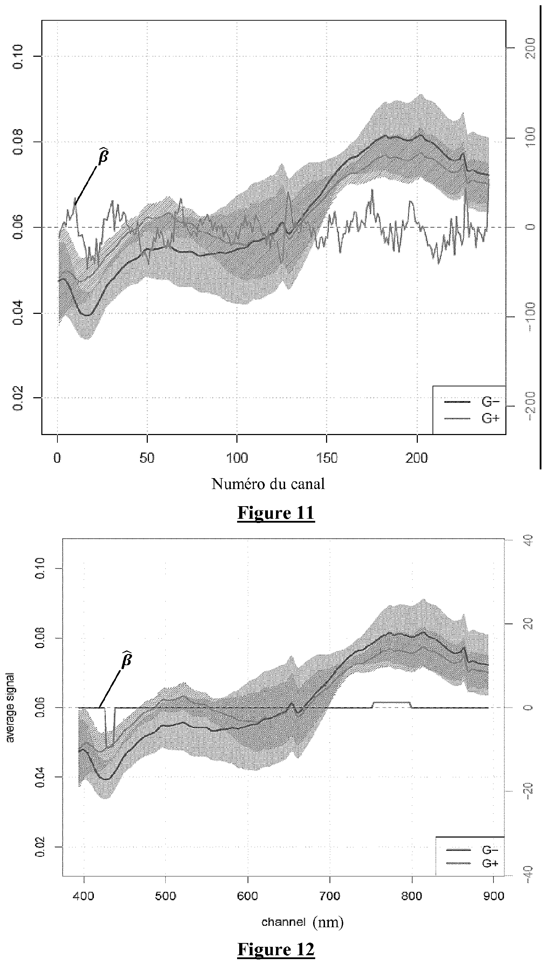

où $\mu_1$ et $\mu_2$ sont deux paramètres réglant respectivement la parcimonie du modèle (i.e. le nombre de composantes de $\hat{\beta}$ qui ne sont pas nulles) et la régularité du modèle (i.e. la variabilité entre deux composantes successives de $\hat{\beta}$). Comme il est visible à la figure 12, lorsque la parcimonie et la régularité sont forcées lors de l'apprentissage de $\hat{\beta}$ ($\mu_1 = 0{,}0001$ et $\mu_2 = 0{,}0025$), les composantes de $\hat{\beta}$ non nulles automatiquement retenues sont celles des gammes 415-440nm et 750-800nm. Le taux de succès est de 100% pour un milieu opaque (e.g. CPSO). Et pour un milieu transparent (e.g. CPSE), le taux de succès est de 92% pour le Gram positif et de 84% pour le Gram négatif.

Il a été décrit une acquisition à l'aide d'une caméra hyperspectrale dans la gamme 400-900nm. Evidemment, l'invention s'applique également à une acquisition réalisée par une caméra multispectrale qui acquière des spectres dans deux gammes séparées comprenant, ou consistant en, respectivement les gammes 415-440nm et 750-800nm. De même, l'invention s'applique à deux caméras distinctes configurées pour respectivement acquérir ces gammes.

Il a été décrit une acquisition dans une gamme plus large que la gamme 415-440nm, notamment une acquisition dans la gamme 750-800nm qui comporte des informations spectrales discriminantes sur le type de Gram des bactéries. En variante, l'acquisition est réalisée uniquement dans la gamme 400-500nm, plus préférentiellement dans la gamme 415-440nm, et la prédiction du type de Gram est réalisée uniquement en fonction des informations spectrales de cette gamme. Evidemment, l'invention s'applique à toute gamme d'acquisition comprenant la gamme 415-440nm, combinée à un traitement uniquement réalisé sur cette gamme pour prédire le type de Gram.

Il a été décrit l'acquisition d'image, c'est-à-dire l'acquisition d'informations spatiales et spectrales. Bien évidemment, l'invention s'applique également à l'acquisition d'informations uniquement spectrales, par exemple à l'aide d'une micro-spectroscopie. Par exemple, une colonie peut être localisée préalablement à l'aide d'un système d'imagerie standard ou par un opérateur, puis un spectre en réflexion ou en transmission émis par la colonie est acquis par micro-spectroscopie et traité pour prédire le type de Gram.

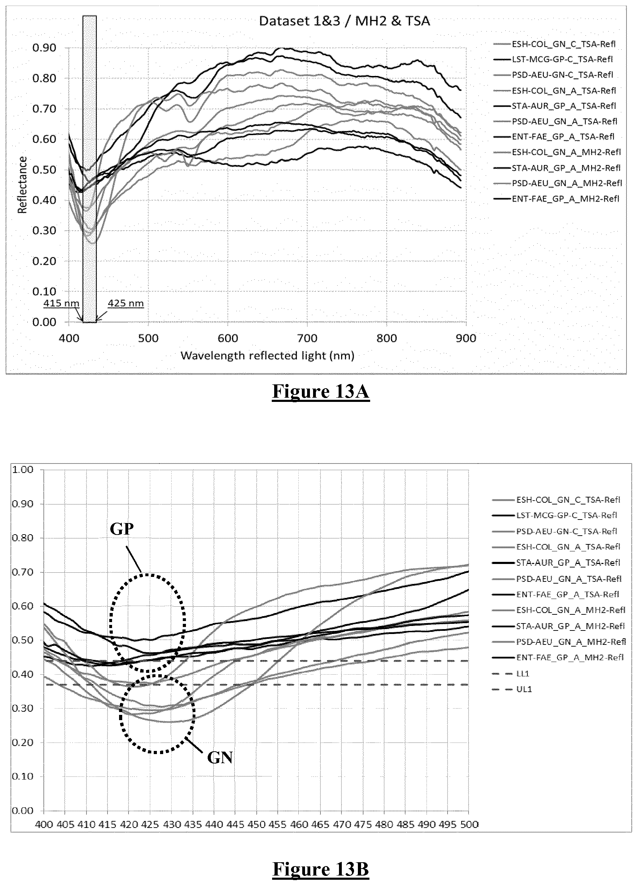

Il a été décrit deux milieux de culture, le CPSE (transparent) et le CPSO (opaque). D'autres milieux de culture ont été testés menant à des résultats analogues. Par exemple sur les figures 13A et 13B, on observe sur un milieu des milieux de culture TSA (gélose trypto-caséine soja) et MH2 (gélose de Mueller Hinton 2) que le Gram positif et le Gram négatif se distinguent l'un de l'autre au moins sur la gamme 415-440nm. Comme décrit précédemment, les milieux opaques sont préférés car ils facilitent la prédiction du type de Gram. L'invention s'applique ainsi à tout type de milieu de culture transparent et non coloré, e.g. ceux de l'état de la technique, auquel est ajouté un opacifiant, par exemple des particules de $SiO_2$.

Il a été décrit différents prédiction (seuillage, modèle linéaire, SVM, Lasso). Bien entendu, l'invention s'applique à tout type de traitement permettant de classer des mesures en deux classes (Gram positif et Gram négatif), par exemple un algorithme de comparaison de spectre (e.g. un méthode de centroïde), un classification à base de réseau de neurones, une classification à base d'arbre (e.g. algoritme CART, pour « classification and regresssion tree »), etc. De telles méthodes sont par exemple celles décrites dans le document d'Eric Laloum, « Une méthode chimiométrique originale d'identification de produits par spectroscopie proche infrarouge », Spectra Analyse, vol.33, n° 237, 2004.

Il a été décrit des modes de réalisation à base de spectre en réflexion. En variante, le spectre est acquis en transmission, les traitements décrits précédemment pour la prédiction du type de Gram s'appliquant.

## Revendications

1. Détection du type de Gram d'une souche bactérienne, comprenant :

   - l'éclairage dans la gamme de longueurs d'onde 415nm-440nm d'au moins une bactérie de ladite souche ayant une réponse électromagnétique naturelle dans ladite gamme;
   - l'acquisition, dans la gamme 415nm-440nm, d'une intensité lumineuse réfléchie par, ou transmise au travers de, ladite bactérie éclairée; et
   - la détermination du type de Gram de la souche bactérienne en fonction de l'intensité lumineuse acquise dans la gamme 415nm-440nm.

2. Détection du type de Gram d'une bactérie selon la revendication 1, dans laquelle ladite bactérie a également une réponse électromagnétique naturelle dans la gamme de longueur d'ondes 750nm-800nm, et dans lequel :

   - l'éclairage de ladite bactérie est également réalisé dans la gamme de longueurs d'onde 750nm-800 nm;
   - l'acquisition d'une intensité lumineuse réfléchie par, ou transmise au travers de, la bactérie éclairée est également réalisé dans la gamme 750-800 nm; et
   - la détermination du type de Gram la souche bactérienne est réalisée en fonction des intensités lumineuses acquises dans lesdites gammes 415nm-440nm et 750nm-800nm.

3. Détection selon la revendication 1 ou 2, dans laquelle la détermination du type de Gram comprend :

   - le calcul d'un facteur de réflectance ou d'absorption de ladite bactérie éclairée en fonction de l'intensité lumineuse acquise ;
   - la détermination du type de Gram en fonction du facteur de réflectance ou d'absorption calculé.

4. Détection selon la revendication 3, dans laquelle la détermination du type de Gram comprend :

   - le calcul d'une dérivée première en la longueur d'onde du facteur de réflectance ou d'absorption calculé ;
   - la détermination du type de Gram en fonction la dérivée première calculée.

5. Détection selon l'une quelconque des revendications précédentes, dans laquelle la détermination du type de Gram est réalisée en fonction de l'intensité lumineuse à une unique longueur d'onde de la gamme 415nm-440nm, en particulier la longueur d'onde 420nm.

6. Détection selon la revendication 5, dans laquelle la détermination du type de Gram comprend :

   - la comparaison d'une valeur égale à l'intensité lumineuse à 420nm, ou calculée à partir de celle-ci, à un premier et second seuils prédéterminés séparant la coloration de Gram positive de la coloration de Gram négative, le premier seuil étant supérieur au second seuil ;
   - la détermination du Gram négatif si ladite valeur est supérieure au premier seuil et la détermination du Gram positif si ladite valeur est inférieure au second seuil.

7. Détection selon l'une quelconque des revendication 1 à 4, dans laquelle la détermination du type de Gram comprend l'application d'une prédiction linéaire prédéterminée reliant l'intensité lumineuse acquise, ou une valeur calculée à partir de celle-ci, au type de Gram, suivie d'un seuillage du résultat de la prédiction linéaire appliquée.

8. Détection selon l'une quelconque des revendications 1 à 4, dans laquelle la détermination du type de Gram comprend l'application d'une classification bi-classe de type SVM à l'intensité lumineuse acquise, ou à une valeur calculée à partir de celle-ci.

9. Détection selon l'une quelconque des revendications précédentes, dans laquelle l'éclairage et l'acquisition sont réalisés directement sur un échantillon comprenant une colonie de le souche bactérienne et un milieu nutritif dans lequel ladite colonie a poussé.

10. Détection selon la revendication 9, dans laquelle la détermination du type de Gram est réalisée en fonction dudit milieu nutritif.

11. Détection selon la revendication 9 ou 10, dans laquelle le milieu nutritif est un substrat opaque à la surface duquel la colonie a poussé, ledit substrat ayant de préférence une réflectance $\rho$ inférieure ou égale à 10%, de préférence inférieure ou égale à 5%.

12. Détection selon l'une quelconque des revendications précédentes, dans laquelle l'acquisition de l'intensité lumineuse comporte l'acquisition d'une image hyperspectrale ou multispectrale d'une colonie de bactérie de la souche, et dans laquelle l'intensité lumineuse est déterminée en fonction d'au moins un pixel de ladite image correspondant à la colonie.

13. Détection selon la revendication 12, dans laquelle l'intensité lumineuse est égale à la moyenne de pixels de ladite image correspondant à la colonie.

14. Système de détection du type de Gram d'une souche bactérienne, comprenant :

- un éclairage configuré pour éclairer, dans la gamme de longueurs d'onde 415nm-440nm, au moins une bactérie de la souche;
- un capteur configuré pour acquérir, dans la gamme 415nm-440nm, une intensité lumineuse réfléchie par, ou transmise au travers de, ladite bactérie éclairée; et
- une unité informatique configurée pour déterminer le type de Gram de la souche bactérienne en fonction de l'intensité lumineuse acquise dans la gamme 415nm-440nm.

15. Système selon la revendication 14, dans lequel :

- l'éclairage est configuré pour éclairer ladite bactérie dans la gamme de longueurs d'onde 750nm-800 nm ;
- le capteur est configuré pour acquérir, dans la gamme de longueurs d'onde 750nm-800 nm, une intensité lumineuse réfléchie par, ou transmise au travers de, ladite bactérie éclairée; et
- l'unité informatique est configurée pour déterminer le type de Gram de la souche bactérienne en fonction de l'intensité lumineuse acquise dans lesdites gammes 415nm-440nm et 750nm-800nm.

16. Système selon la revendication 14 ou 15, configuré pour éclairer, et acquérir l'image de, un échantillon comprenant une colonie de bactéries de ladite souche et milieu nutritif dans lequel ladite colonie a poussé, en particulier une boite de Pétri.

**Patentansprüche**

1. Detektion des Gram-Typs eines Bakterienstamms, umfassend:

- die Beleuchtung, im Wellenlängenbereich von 415 nm bis 440 nm, mindestens einer Bakterie des Stamms mit einer natürlichen elektromagnetischen Reaktion in dem Bereich;
- die Erfassung, im Wellenlängenbereich von 415 nm bis 440 nm, einer Lichtintensität, die von der beleuchteten Bakterie reflektiert oder quer durch diese übertragen wird; und
- die Bestimmung des Gram-Typs des Bakterienstamms als Funktion der Lichtintensität, die im Bereich von 415 nm bis 440 nm erfasst wird.

2. Detektion des Gram-Typs einer Bakterie nach Anspruch 1, wobei die Bakterie auch eine natürliche elektromagnetische Reaktion im Wellenlängenbereich von 750 nm bis 800 nm aufweist, und wobei:

- die Beleuchtung der Bakterie auch in dem Wellenlängenbereich von 750 nm bis 800 nm durchgeführt wird;
- die Erfassung einer Lichtintensität, die von der beleuchteten Bakterie reflektiert oder quer durch diese übertragen wird, auch im Bereich von 750 nm bis 800 nm durchgeführt wird; und
- die Bestimmung des Gram-Typs des Bakterienstamms als Funktion der Lichtintensitäten durchgeführt wird, die in den Bereichen von 415 nm bis 440 nm und 750 nm bis 800 nm erfasst werden.

3. Detektion nach Anspruch 1 oder 2, wobei die Bestimmung des Gram-Typs umfasst:

- die Berechnung eines Reflexions- oder Absorptionsfaktors der beleuchteten Bakterie als Funktion der erfassten Lichtintensität;
- die Bestimmung des Gram-Typs als Funktion des berechneten Reflexions- oder Absorptionsfaktors.

4. Detektion nach Anspruch 3, wobei die Bestimmung des Gram-Typs umfasst:

- die Berechnung einer ersten Ableitung in der Wellenlänge des berechneten Reflexions- oder Absorptionsfaktors;
- die Bestimmung des Gram-Typs als Funktion der berechneten ersten Ableitung.

5. Detektion nach einem der vorhergehenden Ansprüche, wobei die Bestimmung des Gram-Typs als Funktion der Lichtintensität bei einer einzigen Wellenlänge des Bereichs von 415 nm bis 440 nm, insbesondere der Wellenlänge von 420 nm, durchgeführt wird.

6. Detektion nach Anspruch 5, wobei die Bestimmung des Gram-Typs umfasst:

- den Vergleich eines Werts gleich der Lichtintensität bei 420 nm, oder berechnet aus dieser, mit einer ersten und zweiten vorherbestimmten Schwelle, weiche die Färbung von Gram-positiv von der Färbung von Gram-negativ trennen, wobei die erste Schwelle größer ist als die zweite Schwelle;
- die Bestimmung von Gram-negativ, wenn der Wert größer ist als die erste Schwelle, und die Bestimmung von Gram-positiv, wenn der Wert kleiner ist als die zweite Schwelle.

7. Detektion nach einem der Ansprüche 1 bis 4, wobei die Bestimmung des Gram-Typs die Anwendung einer vorherbestimmten linearen Prädiktion, welche die erfasste Lichtintensität, oder einen aus dieser berechneten Wert, mit dem Gram-Typ verbindet, auf den Gram-Typ, gefolgt von einer Schwellenwertbestimmung des Ergebnisses der angewendeten linearen Prädiktion, umfasst.

8. Detektion nach einem der Ansprüche 1 bis 4, wobei die Bestimmung des Gram-Typs die Anwendung einer zweiklassigen Klassifikation vom Typ SVM auf die erfasste Lichtintensität, oder einen aus diesem berechneten Wert, umfasst.

9. Detektion nach einem der vorhergehenden Ansprüche, wobei die Beleuchtung und die Erfassung direkt an einer Probe durchgeführt werden, die eine Kolonie des Bakterienstamms und ein Nährmedium, in dem die Kolonie gewachsen ist, umfasst.

10. Detektion nach Anspruch 9, wobei die Bestimmung des Gram-Typs als Funktion des Nährmediums durchgeführt wird.

11. Detektion nach Anspruch 9 oder 10, wobei das Nährmedium ein opakes Substrat ist, an dessen Oberfläche die Kolonie gewachsen ist, wobei das Substrat vorzugsweise ein Reflexionsvermögen $\rho$ kleiner oder gleich 10 %, vorzugsweise kleiner oder gleich 5 %, aufweist.

12. Detektion nach einem der vorhergehenden Ansprüche, wobei die Erfassung der Lichtintensität die Erfassung eines hyperspektralen oder multispektralen Bilds einer Bakterienkolonie des Stamms umfasst, und wobei die Lichtintensität als Funktion mindestens eines Pixels des Bilds bestimmt wird, das der Kolonie entspricht.

13. Detektion nach Anspruch 12, wobei die Lichtintensität gleich dem Mittelwert der Pixel des Bilds ist, das der Kolonie entspricht.

14. System zur Detektion des Gram-Typs eines Bakterienstamms, umfassend:

- eine Beleuchtung, die ausgelegt ist, im Wellenlängenbereich von 415 nm bis 440 nm, mindestens einer Bakterie des Stamms zu beleuchten;
- einen Sensor, der ausgelegt ist, im Wellenlängenbereich von 415 nm bis 440 nm, eine Lichtintensität zu erfassen, die von der beleuchteten Bakterie reflektiert oder quer durch diese übertragen wird; und
- eine Recheneinheit, die ausgelegt ist, den Gram-Typ des Bakterienstamms als Funktion der Lichtintensität zu bestimmen, die im Bereich von 415 nm bis 440 nm erfasst wird.

15. System nach Anspruch 14, wobei:

- die Beleuchtung ausgelegt ist, die Bakterie im Weilenlängenbereich von 750 nm bis 800 nm zu beleuchten;
- der Sensor ausgelegt ist, im Wellenlängenbereich von 750 nm bis 800 nm, eine Lichtintensität zu erfassen, die von der beleuchteten Bakterie reflektiert oder quer durch diese übertragen wird; und
- die Recheneinheit ausgelegt ist, den Gram-Typ des Bakterienstamms als Funktion der Lichtintensität zu bestimmen, die in den Bereichen von 415 nm bis 440 nm und 750 nm bis 800 nm erfasst wird.

16. System nach Anspruch 14 oder 15, welches ausgelegt ist, eine Probe zu beleuchten und ein Bild dieser zu erfassen, die eine Bakterienkolonie des Stamms und ein Nährmedium umfasst, in dem die Kolonie gewachsen ist, insbesondere eine Petri-Schale.

## Claims

1. Detection of the Gram type of a bacterial strain, comprising:

- lighting in the 415nm-440nm wavelength range at least one bacterium of said strain having a natural electromagnetic response in said wavelength range;
- acquiring, in the 415nm-440nm range, a luminous intensity reflected by, or transmitted through, said illuminated bacterium; and
- determining the Gram type of the bacterial strain based on the acquired luminous intensity acquired in the 415nm-440nm range.

2. Detection of the Gram type of a bacterial strain according to claim 1, wherein said bacterium further has a natural electromagnetic response in the 750nm-800nm wavelength range, and wherein:

- lighting of said bacterium is also carried out in the 750 nm-800nm wavelength range;
- acquiring, in the 750nm-800nm range, a luminous intensity reflected by, or transmitted through, said illuminated bacterium; and
- the determination of the Gram type of the bacterial strain is carried out based on the luminous intensities acquired in said 415nm-440nm and 750nm-800nm ranges.

3. Detection of the Gram type of a bacterial strain according to claim 1 or 2, wherein the determination of the Gram type of the bacterial strain comprises:

- the computation of a reflectance factor or an absorption factor of said illuminated bacterium based on the acquired luminous intensity;
- the determination of the Gram type based on the computed a reflectance factor or the computed absorption factor.

4. Detection of the Gram type of a bacterial strain according to claim 3, wherein the determination of the Gram type comprises:

- the computation of a first derivative in the wavelength of the computed reflectance factor or of the computed absorption factor;
- the determination of the Gram type based on the first derivative.

5. Detection of the Gram type of a bacterial strain according to any one of the preceding claims, wherein the determination of the Gram type is carried out based on the luminous intensity at a unique wavelength in the 415nm-440nm range, in particular the 420nm wavelength.

6. Detection of the Gram type of a bacterial strain according to claim 5, wherein the determination of the Gram type comprises:

- the comparison of a value equal to the luminous intensity at the 420nm wavelength, or computed based thereon, to a first and second predetermined thresholds separating positive gram staining from negative gram staining, the first threshold being greater than the second threshold;
- the determination of the negative Gram if said value is greater than the first threshold and the determination of the positive Gram if said value is less than the second threshold.

7. Detection of the Gram type of a bacterial strain according to any one of claims 1-4, wherein the determination of the Gram type comprises carrying out a predetermined linear prediction linking the acquired luminous intensity, or a value computed based thereon, and the Gram type, followed by a thresholding of the linear prediction result.

8. Detection of the Gram type of a bacterial strain according to any one of claims 1-4, wherein the determination of the Gram type comprises carrying out a two classes classification of the SVM type to the acquired luminous intensity, or to a value computed based thereon.

9. Detection of the Gram type of a bacterial strain according to any one of the preceding claims, wherein the lightning and the acquisition are carried out directly to a sample comprising a bacterial colony of said strain and a nutrient medium in which said colony has grown.

10. Detection of the Gram type of a bacterial strain according to claim 9, wherein the determination of the Gram type is carried out based on the nutrient medium.

11. Detection of the Gram type of a bacterial strain according to claim 9 or 10, wherein the nutrient medium is an opaque medium on the surface of which the colony has grown, said substrate preferably having a reflectance $\rho$ less than or equal to 10%, preferably less than or equal to 5%.

12. Detection of the Gram type of a bacterial strain according to any one of the preceding claims, wherein the acquisition of the luminous intensity comprises the acquisition of a hyperspectral or multispectral image of a bacterial colony of the strain, and wherein the luminous intensity is determined based on at least one pixel of said image corresponding to the colony,

13. Detection of the Gram type of a bacterial strain according to claim 12, wherein the luminous intensity is equal to the average of pixels of said image corresponding to the colony.

14. System for detection the Gram type of a bacterial strain, comprising:

- a lighting configured to illuminate, in the 415nm-440nm wavelength range, at least one bacterium of the strain;
- a sensor configured to acquire, in the 415nm-440nm range, a luminous intensity reflected by, or transmitted through, said illuminated bacteria;
- a computer unit configured to determine the Gram type of the bacterial strain based on the luminous intensity acquired in the 415nm-440nm range.

15. System for detection the Gram type of a bacterial strain according to claim 14, wherein:

- the lighting is configured to illuminate said bacterium in the 750nm-800nm wavelength range;
- the sensor is configured to acquire, in the 750nm-800nm wavelength range, a luminous intensity reflected by, or transmitted through, said illuminated bacteria; and
- the computed unit is configured to determine the Gram type of the bacterial strain based on the luminous intensities acquired in said 415nm-440nm and 750nm-800nm ranges.

16. System for detection the Gram type of a bacterial strain according to claim 14 or 15, configured to illuminate, and acquire the image of, a sample comprising a colony of bacteria of said strain and a nutrient medium in which said colony has grown, in particular a Petri dish.

**Figure 1**

| | |
|---|---|
| Croissance d'une ou plusieurs colonies de la souche bactérienne sur une gélose préférentiellement opaque dans une boite de Pétri | 32 |
| Eclairage à 400-900 nm de la boite de Pétri ouverte et acquisition d'une image hyperspectrale (« $HSI(\lambda)$ ») à 400-900 nm de la lumière réfléchie par la boite de Pétri | 34 |
| Prétraitement de l'image de radiance $HSI(\lambda)$ | 36 |
| Transformation de l'image de radiance $HSI(\lambda)$ en une image de réflectance (« $Y(\lambda)$ ») | 38 |
| Extraction des zones de pixels (« $Col(\lambda)$ ») correspondant aux colonies de bactéries. | 40 |
| Calcul d'une variable de décision (« $X_{col}(\lambda)$ ») à partir de l'image $Y(\lambda)$ et la zone $Col(\lambda)$ | 42 |
| Prédiction du niveau de Gram de la colonie en fonction de la variable de décision $X_{col}(\lambda)$ | 44 |

**Figure 2**

**Figure 3A (CPSE)**

**Figure 3B (CPSE)**

**Figure 4A (CPSO)**

**Figure 4B(CPSO)**

**Figure 5**

**Figure 6**

Figure 7

| Spectral pre-processing | Maximum correlation value | Wavelength $\lambda_c$ (nm) |
|---|---|---|
| Reflectance (Raw) | 0.65 | 428 |
| Absorbance (Log 1/R) | -0.66 | 427.8 |
| **Reflectance – 1ˢᵗ Der (SG)** | 0.83 | 415.3 |
| Reflectance – 2ⁿᵈ Der (SG) | -0.79 | 434.1 |
| Absorbance – 1ˢᵗ Der (SG) | -0.84 | 417.4 |
| Absorbance – 2ⁿᵈ Der (SG) | 0.86 | 434.1 |
| Reflectance SNV | -0.58 | 396.5 |
| Reflectance Area Normalized | 0.76 | 432 |
| Reflectance Vector Normalized | 0.77 | 432 |
| Absorbance Area Normalized | -0.6 | 432 |
| Absorbance Vector Normalized | -0.59 | 432 |
| Reflectance – 1ˢᵗ Der – Vector Norm | 0.8 | 421.5 |
| Reflectance – Vector Norm – 1ˢᵗ Der | 0.83 | 421.5 |
| Reflectance – 1ˢᵗ Der – SNV | 0.8 | 421.5 |

Figure 8

Predicted vs. Reference

**Figure 9**

Regression coefficients

(52 spectra – 240 spectral channels) 28 GN & 24 GP

X-Variables (GRAMS, Factor-7, B0:0.53934)

**Figure 10**

**Figure 11**

**Figure 12**

**Figure 13A**

**Figure 13B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2010077304 A

### Littérature non-brevet citée dans la description

- **BOSOON P et al.** *Transactions of the ASABE,* 2015, vol. 58 (1), 5-16
- **LUNA-PINEDA T et al.** *Proc of SPIE,* 2007, vol. 6554, 65540K1-11
- **GUILLEMOT M et al.** *Proc of SPIE,* 2016, vol. 9887, 98873L1-12
- **K. VARMUZA ; P. FILZMOSER.** Introduction to Multivariate Statistical Analysis in Chemometrics. CRC Press, 2009
- **A. RINNAN ; F. VAN DEN BERG ; S. ENGELSEN.** Review of the most common pre-processing techniques for near-infrared spectra. *Trends in Analytical Chemistry,* 2009, vol. 28 (10
- **A. SAVITZKY ; M.J.E. GOLAY.** Smoothing and differentialtion of data by simplified least squares procédures. *Anal. Chem.,* 1964, vol. 36, 1627-1639
- **ERIC LALOUM.** Une méthode chimiométrique originale d'identification de produits par spectroscopie proche infrarouge. *Spectra Analyse,* 2004, vol. 33 (237